# EUROPEAN PATENT APPLICATION

(11) **EP 4 830 622 A1**
(43) Date of publication of application: **28.10.2026**
(21) Application number: 24907045.9
(22) Date of filing: 20.11.2024
(51) Int. Cl.: A61B 5/107, A61B 5/00, G06T 7/00, G06T 7/60

(54) **INFORMATION PROCESSING DEVICE, INFORMATION PROCESSING METHOD, AND PROGRAM**

(30) Priority: 20.12.2023 JP 2023214462
(71) Applicant: Panasonic Intellectual Property Management Co., Ltd., Kadoma-shi, Osaka 571-0057 (JP)
(72) Inventor: OMAYU, Hiroaki, Kadoma-shi, Osaka 571-0057 (JP); KANAMORI, Yoshiaki, Kadoma-shi, Osaka 571-0057 (JP); SAITO, Kosuke, Kadoma-shi, Osaka 571-0057 (JP); MINAHASHI, Kan, Kadoma-shi, Osaka 571-0057 (JP)
(74) Representative: Novagraaf International SA
(86) International application number: PCT/JP2024/041081
(87) International publication number: WO 2025/134657

(57) **Abstract**

An information processing device (100) evaluates a hair growth-inhibiting effect of a light-based hair removal device (10). The information processing device (100) includes an obtainer (111) that obtains a skin image in which a target portion of a subject person is captured; a counter (112) that counts a total number of body hairs from the skin image (200); and an evaluator (113) that evaluates the hair growth-inhibiting effect, based on a result of comparison between (i) a first count result that is a result of counting performed on a first skin image captured at a first timing and (ii) a predicted hair amount value at the first timing based on a second count result that is a result of counting performed on a second skin image captured at a second timing that precedes the first timing by a first period.

## Description

### [Technical Field]

The present disclosure relates to an information processing device, an information processing method, and a program.

### [Background Art]

Patent Literature (PTL) 1 discloses a measuring system that measures the number of body hairs and evaluates hair removal or hair restoration.

### [Citation List]

### [Patent Literature]

[PTL1] Japanese Unexamined Patent Application Publication No. 2021-56708

### [Summary of Invention]

### [Technical Problem]

The present disclosure provides an information processing device that can appropriately evaluate the hair growth-inhibiting effect of a light-based hair removal device.

### [Solution to Problem]

An information processing device according to an aspect of the present disclosure is an information processing device that evaluates a hair growth-inhibiting effect of a light-based hair removal device. The information processing device includes: an obtainer that obtains a skin image in which a target portion of a subject person is captured; a counter that counts a total number of body hairs from the skin image; and an evaluator that evaluates the hair growth-inhibiting effect, based on a result of comparison between (i) a first count result that is a result of counting performed by the counter on a first skin image captured at a first timing and (ii) a predicted hair amount value at the first timing based on a second count result that is a result of counting performed by the counter on a second skin image captured at a second timing that precedes the first timing by a first period, the first skin image and the second skin image each being the skin image.

Moreover, an information processing method according to an aspect of the present disclosure is an information processing method that is performed by an information processing device that includes one or more processors and evaluates a hair growth-inhibiting effect of a light-based hair removal device. The information processing method includes the following performed by the one or more processors: obtaining a skin image in which a target portion of a subject person is captured; counting a total number of body hairs from the skin image; and evaluating the hair growth-inhibiting effect, based on a result of comparison between (i) a first count result that is a result of the counting performed on a first skin image captured at a first timing and (ii) a predicted hair amount value at the first timing based on a second count result that is a result of the counting performed on a second skin image captured at a second timing that precedes the first timing by a first period, the first skin image and the second skin image each being the skin image.

It should be noted that these general or specific aspects may be realized as a device, an integrated circuit, a computer program, or a non-transitory computer-readable recording medium such as a CD-ROM, or any combination of a device, an integrated circuit, a computer program, and a non-transitory recording medium.

### [Advantageous Effects of Invention]

An information processing device or the like according to the present disclosure can appropriately evaluate the hair growth-inhibiting effect of a light-based hair removal device.

### [Brief Description of Drawings]

[FIG. 1]

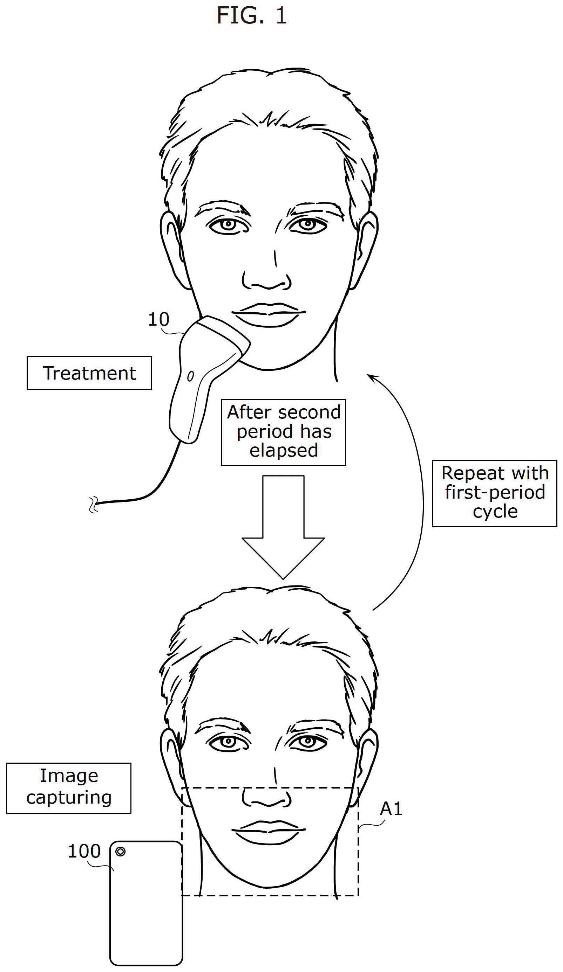

   FIG. 1 is a diagram for describing an overview of an information processing device according to an embodiment.
[FIG. 2]

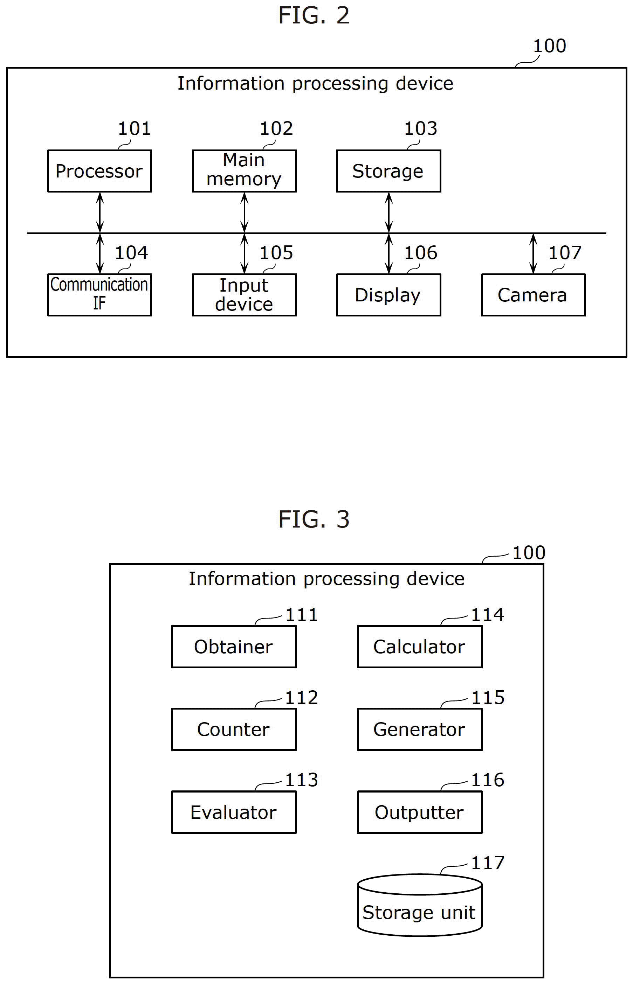

   FIG. 2 is a diagram of a hardware configuration of the information processing device according to the embodiment.
[FIG. 3]
   FIG. 3 is a block diagram illustrating an example of a functional configuration of the information processing device according to the embodiment.
[FIG. 4]

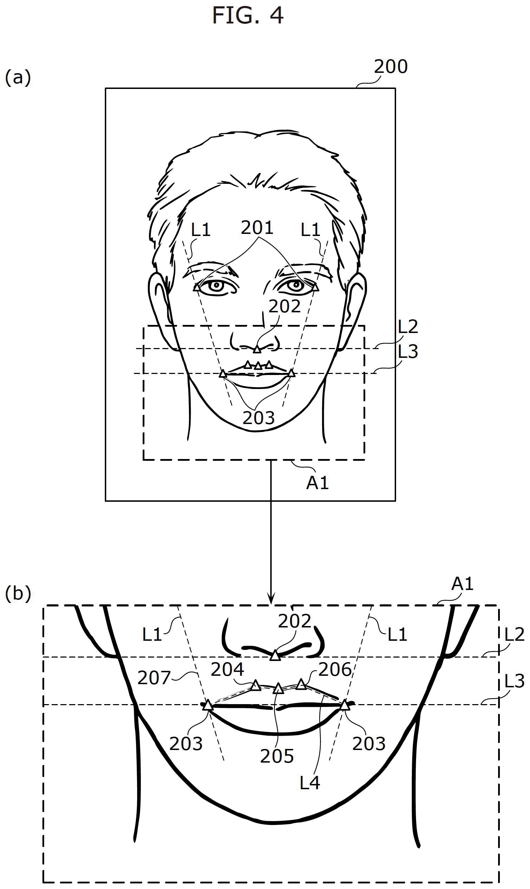

   FIG. 4 is a diagram for describing processing of setting, in a skin image, a region that is a target for counting body hairs.
[FIG. 5]

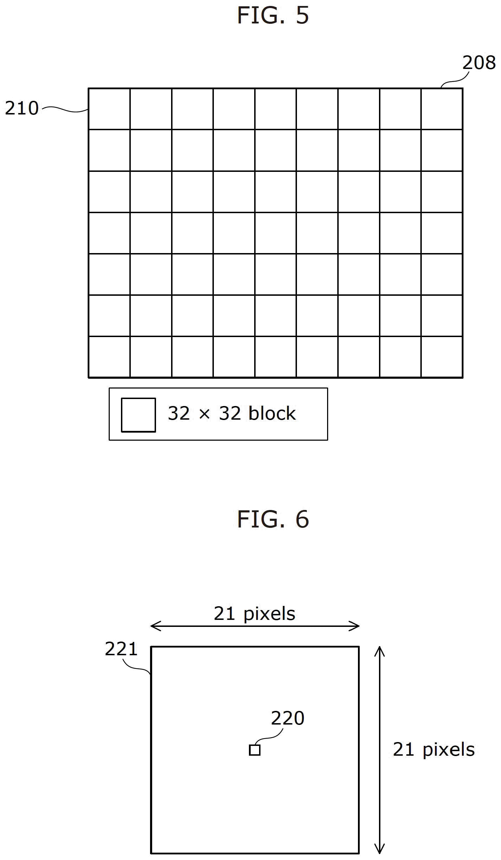

   FIG. 5 is a diagram for describing processing of calculating a constant for determining a threshold value in binarization of a skin image.
[FIG. 6]
   FIG. 6 is a diagram for describing a luminance value used in binarization of a skin image.
[FIG. 7]

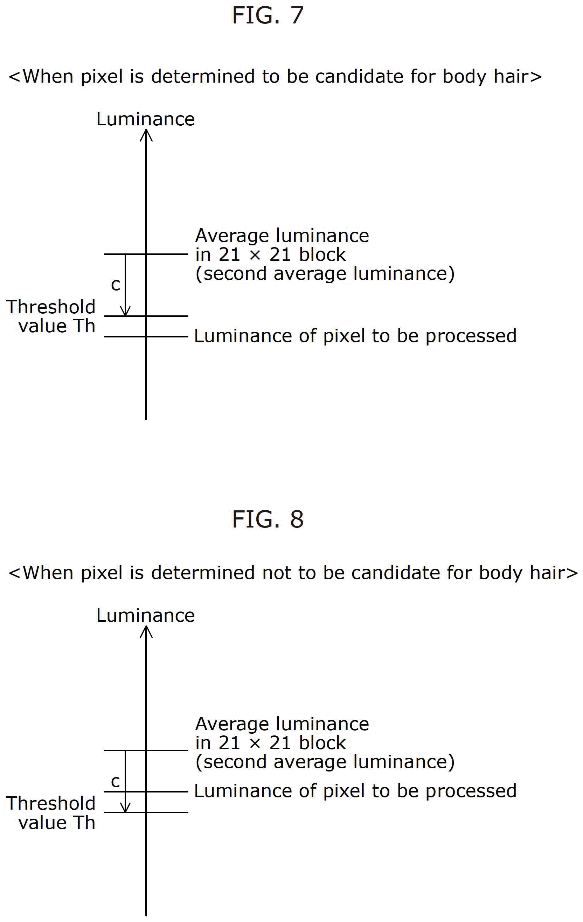

   FIG. 7 is a diagram for determining, in binarization of a skin image, a pixel that is a candidate for body hair.
[FIG. 8]
   FIG. 8 is a diagram for determining, in binarization of a skin image, a pixel that is not a candidate for body hair.
[FIG. 9]

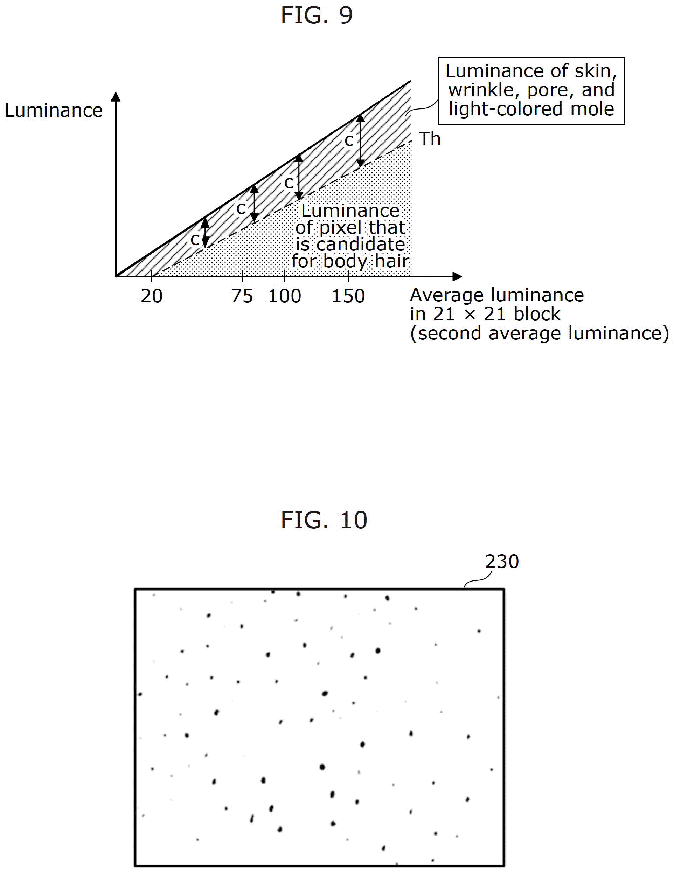

   FIG. 9 is a graph showing the relationship between a second average luminance and a threshold value used for determining a pixel that is a candidate for body hair.
[FIG. 10]
   FIG. 10 is a diagram illustrating an example of a binarized image.
[FIG. 11]

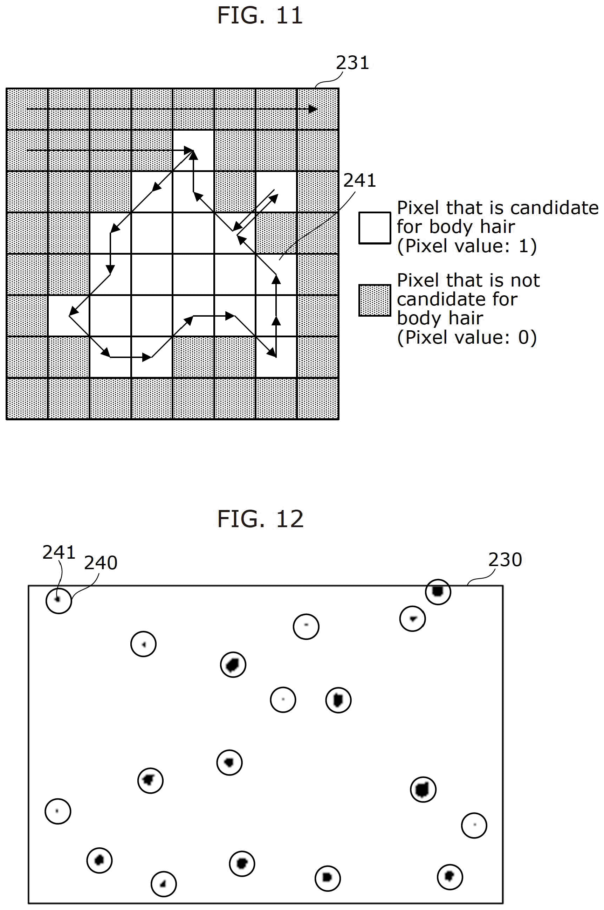

   FIG. 11 is a diagram for describing an example of a pixel group detection method.
[FIG. 12]
   FIG. 12 is a diagram illustrating an example of pixel groups each of which is a cluster of pixels that are candidates for body hair in a binarized image.
[FIG. 13]

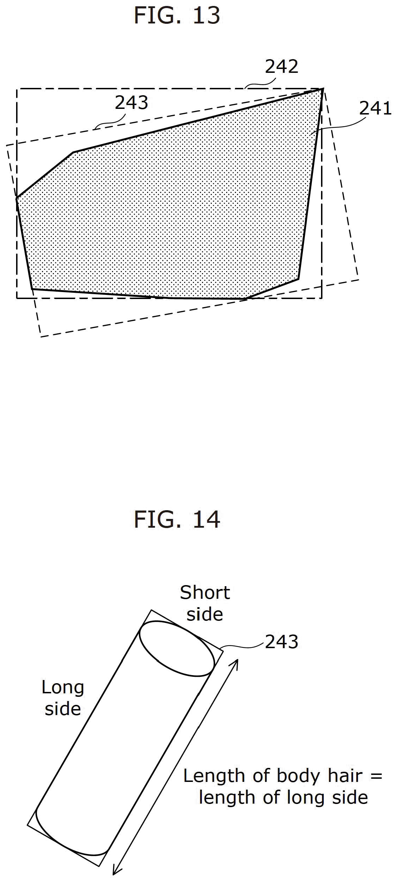

   FIG. 13 is a diagram illustrating an example of rectangles each surrounding a pixel group.
[FIG. 14]
   FIG. 14 is a diagram for describing processing of identifying the length of a body hair.
[FIG. 15]

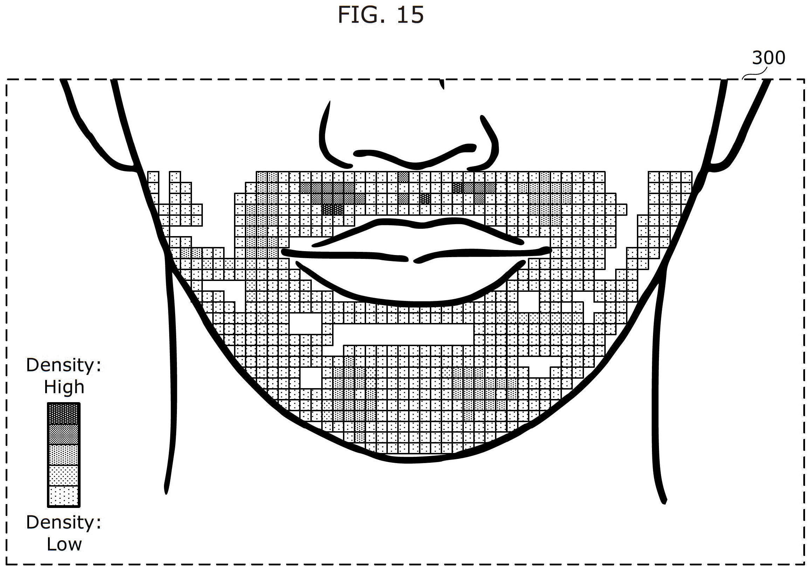

   FIG. 15 is a diagram illustrating an example of heat map data.
[FIG. 16]

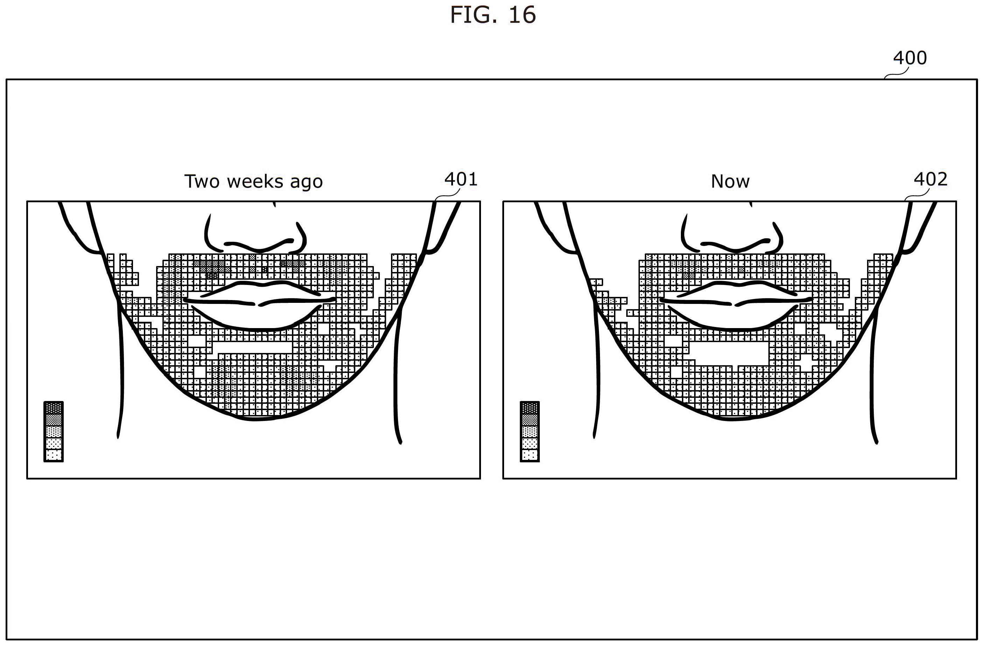

   FIG. 16 is a diagram illustrating an example of display information.
[FIG. 17]

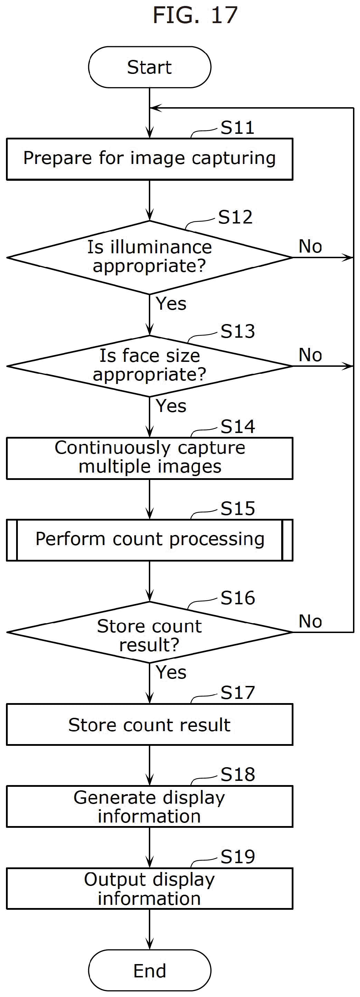

   FIG. 17 is a diagram illustrating an example of processing of capturing a skin image at a second timing (e.g., at the first time) and detecting a hair amount.
[FIG. 18]

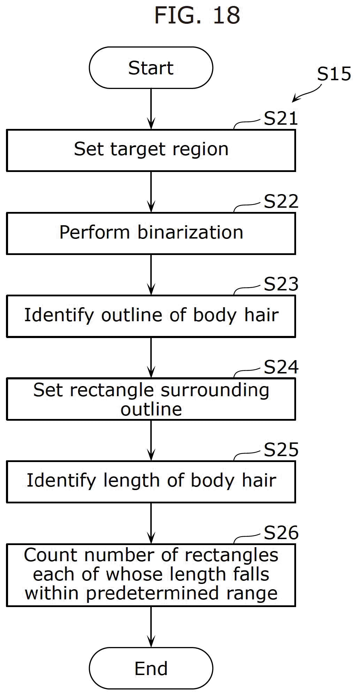

   FIG. 18 is a flowchart illustrating the details of count processing.
[FIG. 19]

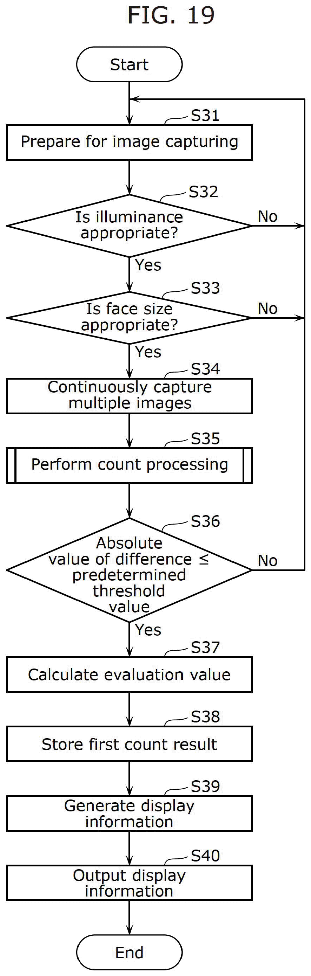

   FIG. 19 is a diagram illustrating an example of processing of capturing a skin image at a first timing (e.g., at the second time or later) and detecting a hair amount.

### [Description of Embodiments]

### (Underlying Knowledge Forming Basis of the Present Disclosure)

Human body hair grows in a certain cycle called a hair cycle. The hair cycle includes an anagen phase, a catagen phase, and a telogen phase, and body hair repeats the anagen phase, the catagen phase, and the telogen phase. A body hair grows in the anagen phase, regresses in the catagen phase, and then transitions to the telogen phase. When a new body hair emerges in the same hair follicle, the body hair in the telogen phase falls out. Accordingly, since the hair cycle includes a phase during which body hair appears outside and a phase during which body hair does not appear outside, it is difficult to distinguish whether body hair does not appear outside due to a hair growth-inhibiting effect or due to the telogen phase. Therefore, the hair growth-inhibiting effect of a light-based hair removal device needs to be evaluated after a period based on the hair cycle elapses from when a treatment using the light-based hair removal device is performed.

Moreover, the hair growth-inhibiting effect varies depending on the individual or the portion on which the treatment has been performed. Accordingly, there may be a case where a change due to the hair growth-inhibiting effect is not visually noticeable but appears little by little. Therefore, it is required to present, to a user, whether the hair growth-inhibiting effect of the light-based hair removal device has been produced.

In the technique disclosed in PTL 1, hair removal or hair restoration is evaluated quantitatively by measuring the number of body hairs.

However, since whether the hair growth-inhibiting effect of a light-based hair removal device has been appropriately produced is unclear in the technique disclosed in PTL 1, it is difficult for a user to determine whether to continue a treatment without adjusting the output level of the light-based hair removal device or to perform the treatment while adjusting the output level. In other words, in the conventional technique, since it is difficult to appropriately set the output of a light-based hair removal device, there is a problem that a user causes unnecessary damage to skin due to an excessively high output or a problem that an appropriate hair growth-inhibiting effect is not produced due to an excessively low output.

In order to solve these problems, the inventors of the present disclosure have found an information processing device or the like that can appropriately evaluate the hair growth-inhibiting effect of a light-based hair removal device.

An information processing device according to a first aspect of the present disclosure is an information processing device that evaluates a hair growth-inhibiting effect of a light-based hair removal device. The information processing device includes: an obtainer that obtains a skin image in which a target portion of a subject person is captured; a counter that counts a total number of body hairs from the skin image; and an evaluator that evaluates the hair growth-inhibiting effect, based on a result of comparison between (i) a first count result that is a result of counting performed by the counter on a first skin image captured at a first timing and (ii) a predicted hair amount value at the first timing based on a second count result that is a result of counting performed by the counter on a second skin image captured at a second timing that precedes the first timing by a first period, the first skin image and the second skin image each being the skin image.

According to this, since comparison is performed between (i) the first count result for the first skin image captured at the first timing and (ii) the predicted hair amount value predicted based on the hair growth-inhibiting effect due to a treatment performed at a timing corresponding to the second timing by the light-based hair removal device, it is possible to determine whether a predicted hair growth-inhibiting effect has been realized. Thus, the hair growth-inhibiting effect of the light-based hair removal device can be appropriately evaluated.

An information processing device according to a second aspect of the present disclosure is the information processing device according to the first aspect, the first timing is a timing after a second period elapses from when a treatment using the light-based hair removal device is performed, and the second timing is a timing after the second period elapses from when an other treatment is performed subsequent to the treatment using the light-based hair removal device.

Hence, since the first timing and the second timing can each be a timing after the second period elapses from when a corresponding treatment is performed, the hair growth-inhibiting effect can be evaluated in a way that reduces an error due to a body hair growth amount.

An information processing device according to a third aspect of the present disclosure is the information processing device according to the first or second aspect, and when an absolute value of a value obtained by subtracting the first count result from the predicted hair amount value is less than or equal to a predetermined threshold value, the evaluator calculates, based on a difference between the first count result and the predicted hair amount value, an evaluation value indicating the hair growth-inhibiting effect, and outputs the evaluation value calculated.

According to this, since the first count result obtained can be determined to be appropriate when the absolute value of the value obtained by subtracting the first count result from the predicted hair amount value is less than or equal to the predetermined threshold value, the appropriate evaluation value can be calculated.

An information processing device according to a fourth aspect of the present disclosure is the information processing device according to any one of the first to third aspects, and when the evaluation value is greater than the predetermined threshold value, the evaluator outputs presentation information for prompting the subject person to capture a new skin image of the subject person.

According to this, since image capturing can be determined to have failed when the evaluation value is greater than the predetermined threshold value, an appropriate first count result can be obtained by prompting image recapturing.

An information processing device according to a fifth aspect of the present disclosure is the information processing device according to any one of the first to fourth aspects, and the counter: for each of a plurality of body hairs in the skin image, (i) extracts an outline of the body hair and (ii) sets a rectangle that surrounds the outline extracted and has a minimum area; and counts, as the total number of body hairs, a total number of one or more rectangles each having at least one of a long side whose length falls within a predetermined range or a short side whose length falls within the predetermined range, among the rectangles set.

Hence, since a portion other than body hair, such as a skin wrinkle, a pore, or a mole, can be excluded from counting, the number of body hairs can be appropriately counted.

An information processing device according to a sixth aspect of the present disclosure is the information processing device according to the fifth aspect, and the counter sets, between a nose and an upper lip of the subject person in the skin image, a target region that is a target for body hair detection, and sets the rectangles in the target region.

Hence, a facial hair can be appropriately counted as a body hair appearing on a human face.

An information processing device according to a seventh aspect of the present disclosure is the information processing device according to any one of the first to sixth aspects. The information processing device according to the seventh aspect further includes: a calculator that divides, into a plurality of divided regions, a skin region that is a region of the target portion in the skin image, and calculates a body hair density for each of the plurality of divided regions; a generator that generates heat map data indicating the body hair densities in the skin region, based on a result of calculation performed by the calculator; and an outputter that outputs display information for displaying first heat map data obtained from the first skin image and second heat map data obtained from the second skin image in a state where the first heat map data and the second heat map data can be compared with each other, the first heat map data and the second heat map data each being the heat map data.

Hence, the distribution of the body hair densities can be visualized.

An information processing method according to an eighth aspect of the present disclosure is an information processing method that is performed by an information processing device that includes one or more processors and evaluates a hair growth-inhibiting effect of a light-based hair removal device. The information processing method includes the following performed by the one or more processors: obtaining a skin image in which a target portion of a subject person is captured; counting a total number of body hairs from the skin image; and evaluating the hair growth-inhibiting effect, based on a result of comparison between (i) a first count result that is a result of the counting performed on a first skin image captured at a first timing and (ii) a predicted hair amount value at the first timing based on a second count result that is a result of the counting performed on a second skin image captured at a second timing that precedes the first timing by a first period, the first skin image and the second skin image each being the skin image.

According to this, since comparison is performed between (i) the first count result for the first skin image captured at the first timing and (ii) the predicted hair amount value predicted based on the hair growth-inhibiting effect due to a treatment performed at a timing corresponding to the second timing by the light-based hair removal device, it is possible to determine whether a predicted hair growth-inhibiting effect has been realized. Thus, the hair growth-inhibiting effect of the light-based hair removal device can be appropriately evaluated.

A program according to a ninth aspect of the present disclosure is a program for causing a computer to execute the information processing method according to the eighth aspect.

It should be noted that these general or specific aspects may be realized as a device, an integrated circuit, a computer program, a non-transitory computer-readable recording medium such as a CD-ROM, or any combination of a device, an integrated circuit, a computer program, and a non-transitory recording medium.

Hereinafter, an embodiment will be described in detail with reference to the Drawings as necessary. However, there are instances where excessively detailed description is omitted. For example, details regarding known facts or duplicate explanation of two or more essentially identical configurations may be omitted. This is to avoid redundancy and to make the following description easier for those skilled in the art to understand.

It should be noted that the inventors have provided the attached drawings and the following description to facilitate sufficient understanding of the present disclosure by those skilled in the art, and thus the attached drawings and the following description are not intended to limit the scope of the subject matter recited in the claims.

### (Embodiment)

### [Configuration]

FIG. 1 is a diagram for describing an overview of an information processing device according to an embodiment.

Information processing device 100 is a device for evaluating the hair growth-inhibiting effect of light-based hair removal device 10. Light-based hair removal device 10 is a device that illuminates skin with intense pulsed light (IPL). Accordingly, light-based hair removal device 10 can damage a hair matrix cell or a hair papilla in skin illuminated with IPL. Thus, the hair growth-inhibiting effect of inhibiting body hair from growing can be obtained.

For example, after a user (subject person) shaves body hair (e.g., facial hair) in a target portion on skin by using a razor or an electric shaver (i.e., after shaving), the user performs a treatment of illuminating the skin with IPL by using light-based hair removal device 10. The timing of the treatment is, for example, immediately after the shaving. In other words, the timing of the shaving and the timing of the treatment are almost the same, and it can be said that these timings correspond to each other.

Then, after a second period (e.g., 1 to 1.5 days) has elapsed from the treatment, the user captures, using information processing device 100, an image of the target portion on which the treatment has been performed. It should be noted that the reason why the second period is set to 1 to 1.5 days is that, when 0.5 days have elapsed after shaving, it is difficult to detect a growing body hair from a skin image since there may be a body hair that has not yet appeared on the surface of the skin, and when 2 or more days have elapsed after shaving, it is difficult to count body hairs since adjacent body hairs begin to intersect. In other words, when the second period is set to 1 to 1.5 days, it is easy to detect and count a growing body hair from a skin image.

The user repeats, with a first-period cycle, a treatment using light-based hair removal device 10 and image capturing using information processing device 100. Accordingly, a plurality of skin images captured at mutually different timings are stored in information processing device 100. The first period is set to 2 to 8 weeks, for example. Information processing device 100 evaluates the hair growth-inhibiting effect of the light-based hair removal device by performing image processing on the plurality of skin images captured at the mutually different timings.

It should be noted that the first period may be varied based on the elapsed time from the initial treatment using light-based hair removal device 10. For example, when the elapsed time is 0 to 2 months, the first period may be set to 2 weeks, and when the elapsed time is 3 or more months, the first period may be set to 4 to 8 weeks. Thus, the first period may be set so that the first period becomes longer as the elapsed time becomes longer.

FIG. 2 is a diagram of a hardware configuration of the information processing device according to the embodiment.

As illustrated in FIG. 2, as the hardware configuration, information processing device 100 includes processor 101, main memory 102, storage 103, communication interface (IF) 104, input device 105, display 106, and camera 107.

Processor 101 is a processor that executes a program stored in storage 103 or the like.

Main memory 102 is a volatile storage region that is: used for temporarily storing data generated through processing performed by processor 101; used as a work area to be used when processor 101 executes a program; or used for temporarily storing data received by communication IF 104.

Storage 103 is a non-volatile storage region that stores various data such as a program. Storage 103 stores, for example: various data generated as a result of processing performed by processor 101; or various data received by communication IF 104.

Communication IF 104 is a communication interface for transmitting and receiving information to and from an other information processing device via a network not shown in the Drawings. Communication IF 104 may be, for example, an interface for wireless communication, such as a wireless LAN interface or a Bluetooth (registered trademark) interface. Communication IF 104 may be an interface for wired communication, such as a universal serial bus (USB) or a wired LAN interface.

Input device 105 is an interface for receiving an input from a person. Input device 105 may be a keyboard or a pointing device such as a mouse, a touchpad, a touch panel, or a trackball.

Display 106 is a liquid crystal display, an organic electroluminescence (EL) display, or the like.

Camera 107 generates an image including a subject, by capturing an image of the subject. Camera 107 may be a color camera (RGB camera), a monochrome camera, or the like.

Information processing device 100 may be, for example, a mobile terminal such as a smartphone or a tablet terminal, or may be a personal computer (PC).

FIG. 3 is a block diagram illustrating an example of a functional configuration of the information processing device according to the embodiment.

As illustrated in FIG. 3, as the functional configuration, information processing device 100 includes obtainer 111, counter 112, evaluator 113, calculator 114, generator 115, outputter 116, and storage unit 117. Information processing device 100 does not necessarily include calculator 114, generator 115, and outputter 116.

Obtainer 111 obtains a skin image in which a target portion of a user (subject person) is captured. Obtainer 111 obtains the skin image by capturing an image of the target portion. Obtainer 111 obtains a skin image each time image capturing is performed. In other words, obtainer 111 may obtain a plurality of skin images captured at mutually different timings. Obtainer 111 obtains a plurality of skin images at different timings since each skin image is obtained each time image capturing is performed. A skin image obtained by obtainer 111 is outputted to counter 112 and storage unit 117. Obtainer 111 is, for example, realized by camera 107.

Counter 112 performs count processing of counting the number of body hairs from a skin image. Each time a skin image is obtained by obtainer 111, counter 112 performs the count processing on the skin image obtained. In other words, counter 112 performs the count processing on each of a plurality of skin images. Counter 112 is, for example, realized by processor 101, main memory 102, storage 103, and the like.

Evaluator 113 performs comparison between (i) a first count result that is a result of the count processing performed on a first skin image and (ii) a predicted hair amount value based on a second count result that is a result of the count processing performed on a second skin image. Among the plurality of skin images, the first skin image is a skin image generated at a first timing by obtainer 111. In other words, the first skin image is a skin image captured at the first timing. Among the plurality of skin images, the second skin image is a skin image generated at a second timing by obtainer 111. In other words, the second skin image is a skin image captured at the second timing. The second timing is a timing that precedes the first timing by the first period. It should be noted that the first timing is a timing after the second period elapses from when a treatment using light-based hair removal device 10 is performed. Moreover, the second timing is a timing after the second period elapses from when a treatment is performed subsequent to the treatment using light-based hair removal device 10 (the treatment performed at a timing preceding the first timing by the second period).

The predicted hair amount value is a predicted value of a hair amount at the first timing, and is calculated based on the second count result obtained from the second skin image captured at the second timing. In other words, the predicted hair amount value is a hair amount (the number of body hairs) at the first timing that is predicted to be decreased by the hair growth-inhibiting effect expected from the treatment performed at a timing corresponding to the second timing. For example, the predicted hair amount value is calculated by multiplying the second count result by a first ratio smaller than 1 (e.g., 85%). The predicted hair amount value is calculated assuming that the second count result decreases at a predetermined decrease rate (e.g., 15%). The predetermined decrease rate is a value obtained by subtracting the first ratio from 1.

It should be noted that the predicted hair amount value may be calculated taking the frequency of treatment into account. In such a case, the first ratio used in calculation of the predicted hair amount value may be determined so that the higher the frequency of treatment becomes, the smaller the first ratio becomes.

It should be noted that the first count result is the number of body hairs included in the target portion in the first skin image, and the second count result is the number of body hairs included in the target portion in the second skin image. The first skin image is an image in which the target portion of the user has been captured at the first timing, and the second skin image is an image in which the target portion has been captured at the second timing. In other words, the first skin image and the second skin image have in common the fact that both have captured the same target portion of the user. Moreover, the first count result and the second count result have in common the fact that both indicate the number of body hairs in the target portion. Furthermore, the predicted hair amount value indicates a predicted number of body hairs since the predicted hair amount value is calculated by multiplying the number of body hairs included in the second skin image by the first ratio.

Then, evaluator 113 evaluates the hair growth-inhibiting effect based on a result of the comparison between the first count result and the predicted hair amount value. After the comparison between the first count result and the predicted hair amount value is performed, when the first count result and the predicted hair amount value are close, evaluator 113 calculates an evaluation value indicating the hair growth-inhibiting effect, based on the difference between the first count result and the predicted hair amount value, and outputs the evaluation value calculated. The difference between the first count result and the predicted hair amount value may be, for example, the absolute value of the difference (i.e., the differential) between the predicted hair amount value and the first count result. It should be noted that the difference between the first count result and the predicted hair amount value may be the ratio (i.e., proportion) of the first count result to the predicted hair amount value. Moreover, when the absolute value of a value obtained by subtracting the first count result from the predicted hair amount value is less than or equal to a first number of body hairs, or when the ratio of the first count result to the predicted hair amount value is within the range of ± a second ratio, the first count result and the predicted hair amount value may be determined to be close.

The evaluation value may be the difference calculated itself, or may be a value correlated with the magnitude of the difference. When the difference is a differential, the evaluation value may be calculated so that the evaluation indicated by the evaluation value becomes higher as the differential decreases. When the difference is a proportion, the evaluation value may be calculated so that the evaluation indicated by the evaluation value becomes higher as the proportion becomes closer to 1. In other words, the evaluation value is calculated so that the evaluation indicated by the evaluation value becomes higher as the first count result becomes closer to the predicted hair amount value.

Moreover, when the absolute value of the difference between the first count result and the predicted hair amount value (hereinafter, simply referred to as "absolute value") is greater than a predetermined threshold value, evaluator 113 outputs presentation information for prompting the user to capture (i.e., recapture) a new skin image of the user. For example, the absolute value may be greater than the predetermined threshold value when the first count result (i.e., the number of body hairs detected from the first skin image) is greater than the predicted hair amount value (the number of body hairs predicted based on the number of body hairs detected from the second skin image) by at least a second number of body hairs. The second number of body hairs is a value greater than the first number of body hairs. Evaluator 113 may output the presentation information by causing outputter 116 to display the presentation information, for example. The presentation information includes, for example, a message that prompts the user to recapture a skin image. Thus, by causing information processing device 100 to display the presentation information, it is possible to prompt the user to recapture a skin image of the user using information processing device 100.

It should be noted that the reason why image recapturing is prompted when the absolute value is greater than the predetermined threshold value is as follows. The reason is that, although the hair amount at the first timing is predicted to be smaller than at the second timing in view of the hair growth-inhibiting effect exhibited by light-based hair removal device 10, the absolute value is greater than the predetermined threshold value, that is, the first count result is greater than the predicted hair amount value by at least the second number of body hairs. In other words, the reason is that the absolute value greater than the predetermined threshold value is presumed to result from a failure in image capturing.

Calculator 114 divides, into a plurality of divided regions, a skin region that is a region of the target portion in a skin image, and calculates a body hair density for each of the plurality of divided regions.

Generator 115 generates heat map data indicating the body hair densities in the skin region, based on the result of calculation performed by calculator 114.

Outputter 116 outputs display information for displaying first heat map data obtained from the first skin image and second heat map data obtained from the second skin image in a state where the first heat map data and the second heat map data can be compared with each other. Outputter 116 outputs the display information by displaying the display information. Outputter 116 may output the display information by transmitting the display information to an other information processing device. In such a case, when the other information processing device receives the display information from information processing device 100, the other information processing device may display the display information on a display included in the other information processing device.

Storage unit 117 stores a plurality of skin images obtained by obtainer 111. Storage unit 117 may store a skin image together with time information indicating the timing at which the skin image is captured. Storage unit 117 stores a plurality of skin images, each of which is stored when obtained. The time information may indicate an absolute time or a time relative to a predetermined reference time, as long as the time information indicates the timing at which the skin image has been captured. Moreover, storage unit 117 may store the result of counting performed by counter 112 each time counter 112 performs counting.

### [Details of Count Processing]

Next, the details of the count processing performed by counter 112 will be described with reference to FIG. 4 to FIG. 14.

FIG. 4 is a diagram for describing processing of setting, in a skin image, a region that is a target for counting body hairs. (a) of FIG. 4 shows skin image 200 including the entire face of a user, and (b) of FIG. 4 is an enlarged view of region A1, which includes the vicinity of the mouth of the user in skin image 200.

Counter 112 sets, between the nose and the upper lip of the user in skin image 200, target region 207 that is a target for body hair detection. In other words, counter 112 performs body hair detection in target region 207. Meanwhile, counter 112 does not perform body hair detection in a region other than target region 207.

In order to set target region 207, counter 112 detects feature points 201 to 206 in the face of the user in skin image 200 and sets straight lines L1 to L3 and polygonal line L4 that pass through feature points 201 to 206. Then, counter 112 sets, as target region 207, a region surrounded by straight lines L1 to L3 and polygonal line L4.

Two feature points 201 are feature points that correspond to the positions of the outer corners of the eyes of the user. Feature point 202 is a feature point that corresponds to the position of the center, in the left-right direction, of the lower end of the nose of the user. Two feature points 203 are feature points that correspond to the positions of the both ends, in the left-right direction, of the lip of the user. Two feature points 204 and 206 are feature points that correspond to the peaks of the upper lip of the user. Feature point 205 is a feature point that corresponds to the position of the center, in the left-right direction, of the upper lip of the user.

One of two straight lines L1 is a straight line that passes through, among two feature points 201 and two feature points 203, feature point 201 and feature point 203 located on the left side of the face, and the other of two straight lines L1 is a straight line that passes through, among two feature points 201 and two feature points 203, feature point 201 and feature point 203 located on the right side of the face. Straight line L2 is a straight line that passes through feature point 202 and extends in the left-right direction. Straight line L3 is a straight line that passes through two feature points 203. Polygonal line L4 is a polygonal line that includes a plurality of line segments each of which connects two adjacent feature points among feature points 203 to 206. The plurality of line segments include a line segment connecting feature point 203 on the right side of the lip and feature point 204, a line segment connecting feature point 204 and feature point 205, a line segment connecting feature point 205 and feature point 206, and a line segment connecting feature point 206 and feature point 203 on the left side of the lip.

Target region 207 may be, for example, a region surrounded by two straight lines L1, straight line L2, and polygonal line L4, among straight lines L1 to L3 and polygonal line L4. In such a case, straight line L3 is not necessarily set. Moreover, target region 207 is not limited to the above-described region, and may be, for example, a region surrounded by two straight lines L1, straight line L2, and straight line L3, among straight lines L1 to L3 and polygonal line L4. In such a case, polygonal line L4 is not necessarily set and feature points 204 to 206 are not necessarily detected.

Next, counter 112 binarizes the skin image for body hair detection. Specifically, counter 112 binarizes the skin image by setting 1 as the pixel value of a pixel that is a candidate for body hair in the skin image and 0 as the pixel value of a pixel that is not a candidate for body hair. It should be noted that values set as the pixel values in binarization are not limited to 0 and 1, and other values may be set as the pixel values. Moreover, counter 112 may set 0 as the pixel value of a pixel that is a candidate for body hair and 1 as the pixel value of a pixel that is not a candidate for body hair.

FIG. 5 is a diagram for describing processing of calculating a constant for determining a threshold value in binarization of a skin image.

Counter 112 sets blocks 210 for target region 207. FIG. 5 shows an example in which blocks 210 are set for region 208 that is part of target region 207. Hereinafter, region 208 will be described as an example.

Blocks 210 are set by dividing region 208. In FIG. 5, region 208 is divided into 7 × 9 blocks 210. Each of blocks 210 may be a 32 × 32 block having a size of 32 pixels in the vertical direction and 32 pixels in the horizontal direction, for example. It should be noted that the size of block 210 is not limited to the above-described size, and may be another size such as a 16 × 16 block. For each of blocks 210, counter 112 calculates constant c corresponding to a first average luminance that is the average of luminance values of 32 × 32 pixels in block 210, and sets constant c for block 210. Constant c is used for determining threshold value Th in binarization of the skin image. Constant c is, for example, calculated based on relationship information indicating the relationship between the first average luminance and constant c. The relationship information may be represented by a table or a relational expression, for example. The relationship indicated by the relationship information is, for example, the relationship in which constant c increases as the first average luminance increases. The relationship information is stored in storage unit 117. Counter 112 reads out the relationship information from storage unit 117 and calculates constant c using the first average luminance and the relationship indicated by the relationship information read out.

FIG. 6 is a diagram for describing a luminance value used in binarization of a skin image. FIG. 7 is a diagram for determining, in binarization of a skin image, a pixel that is a candidate for body hair. FIG. 8 is a diagram for determining, in binarization of a skin image, a pixel that is not a candidate for body hair.

Counter 112 performs binarization processing for each of a plurality of pixels (e.g., all pixels) of a skin image. As illustrated in FIG. 6, counter 112 identifies 21 × 21 block 221 that has a size of 21 pixels in the vertical direction and 21 pixels in the horizontal direction and includes, at its center, processing-target pixel 220. Then, counter 112 calculates a second average luminance that is the average of luminance values of 21 × 21 pixels included in block 221 identified.

Counter 112 calculates threshold value Th by subtracting, from the second average luminance of 21 × 21 block 221 including processing-target pixel 220, constant c set for block 210 including processing-target pixel 220. Then, counter 112 determines whether the luminance of processing-target pixel 220 is less than threshold value Th. As illustrated in FIG. 7, when the luminance of processing-target pixel 220 is less than threshold value Th, counter 112 determines that processing-target pixel 220 is a pixel that is a candidate for body hair. According to this, counter 112 sets, for processing-target pixel 220, a pixel value (e.g., 1) indicating that the pixel is a candidate for body hair. Moreover, as illustrated in FIG. 8, when the luminance of processing-target pixel 220 is greater than or equal to threshold value Th, counter 112 determines that processing-target pixel 220 is a pixel that is not a candidate for body hair. According to this, counter 112 sets, for processing-target pixel 220, a pixel value (e.g., 0) indicating that the pixel is not a candidate for body hair. Thus, counter 112 performs binarization of target region 207 in skin image 200.

FIG. 9 is a graph showing the relationship between a second average luminance and a threshold value for determining a pixel that is a candidate for body hair.

As illustrated in FIG. 9, threshold value Th is set according to the second average luminance, a pixel having a pixel value less than threshold value Th is a pixel that is a candidate for body hair, and a pixel having a pixel value greater than or equal to threshold value Th is a pixel that is not a candidate for body hair. A pixel that is not a candidate for body hair is considered to be, for example, a pixel of a portion other than body hair, such as skin, a wrinkle, a pore, or a light-colored mole.

Counter 112 generates binarized image 230 as illustrated in FIG. 10 by performing binarization processing on target region 207 in skin image 200. FIG. 10 is a diagram illustrating an example of the binarized image.

Next, counter 112 detects, from binarized image 230, a pixel group consisting of a plurality of pixels that are candidates for body hair and are adjacent to each other. In other words, counter 112 detects, as a pixel group, a cluster of pixels that are candidates for body hair.

FIG. 11 is a diagram for describing an example of a pixel group detection method. In FIG. 11, region 231 that is part of binarized image 230 is shown.

Counter 112 searches for, among the plurality of pixels constituting binarized image 230, a pixel whose pixel value is 1 while performing, for example, a raster scan from the upper-left pixel as indicated by the arrow in the horizontal direction in FIG. 11. Then, when counter 112 finds a pixel whose pixel value is 1, counter 112 searches for, in the counter-clockwise direction, a pixel whose pixel value is 1 among unsearched pixels, starting from the pixel located at the lower-left of the pixel found to have a pixel value of 1. By repeating the search until the search returns to the pixel that has been found to have a pixel value of 1 first, the pixels of the outline of the pixel group, each of which has a pixel value of 1, are identified. Accordingly, as illustrated in FIG. 12, counter 112 detects, in binarized image 230, pixel groups 241 each of which consists of pixels each having a pixel value of 1. Each pixel group 241 is indicated by a region that is represented in black and surrounded by circle 240 in FIG. 12. FIG. 12 is a diagram illustrating an example of detection of pixel groups each of which is a cluster of pixels that are candidates for body hair in a binarized image.

Next, for each of the plurality of pixel groups detected, counter 112 sets a rectangle that surrounds the outline of the pixel group (i.e., the outline of the body hair) and has the minimum area. FIG. 13 is a diagram illustrating an example of rectangles each surrounding a pixel group. As illustrated in FIG. 13, counter 112 may set rectangles 242 and 243 each surrounding pixel group 241, and may set, as a rectangle that surrounds pixel group 241 and has the minimum area, rectangle 243 that has the minimum area among rectangles 242 and 243.

As described with reference to FIG. 11 to FIG. 13, for each of the plurality of body hairs in skin image 200, counter 112 (i) extracts the outline of the body hair and (ii) sets a rectangle that surrounds the outline extracted and has the minimum area.

Next, as illustrated in FIG. 14, counter 112 identifies, as the length of the body hair, the long side of rectangle 243 set. FIG. 14 is a diagram for describing processing of identifying the length of a body hair. The length of a body hair is indicated by the number of pixels corresponding to the long side. Then, counter 112 counts, as the number of body hairs, the number of one or more rectangles each of which has the length of the body hair identified (i.e., the length of the long side) that falls within a predetermined range, among the plurality of rectangles set for the plurality of pixel groups. The predetermined range is, for example, a range indicated by the number of pixels that is greater than threshold value Th1 and less than or equal to threshold value Th2. Threshold value Th1 is a value that is less than threshold value Th2 and represents the number of pixels. For example, threshold value Th1 may be 1 and threshold value Th2 may be 100. These threshold values Th1 and Th2 are an example of setting values when an image capturing distance is 20 cm and one pixel corresponds to 0.06 mm, for example. In other words, threshold value Th1 and threshold value Th2 are not limited to the above-described examples as long as threshold value Th1 is set to the number of pixels corresponding to 0.06 mm and threshold value Th2 is set to the number of pixels corresponding to 6 mm.

### [Details of Processing of Generating Heat Map Data]

Next, the details of processing of generating heat map data performed by calculator 114 and generator 115 will be described.

The result of counting performed by counter 112 may include the position (two-dimensional position) of each of one or more rectangles identified as one or more body hairs in skin image 200. For each of a plurality of divided regions into which a skin region that is a region of a target portion in a skin image has been divided, calculator 114 counts one or more rectangles identified as one or more body hairs included in the divided region, using the result of counting performed by counter 112. Thus, calculator 114 calculates a body hair density for each of the plurality of divided regions. When the plurality of divided regions have the same area, the body hair density of each of the plurality of divided regions may be the number of one or more rectangles identified as one or more body hairs included in the divided region. When the plurality of divided regions have mutually different areas, the body hair density of each of the plurality of divided regions may be a value obtained by dividing, by the area of the divided region, the number of one or more rectangles identified as one or more body hairs included in the divided region.

Generator 115 classifies, into any one of a plurality of levels, the body hair density of each of the plurality of divided regions calculated by calculator 114, and displays each of the plurality of divided regions in a display mode indicating the level classified. As illustrated in FIG. 15, generator 115 generates, for example, heat map data 300 in which each of the plurality of divided regions is classified into any one of five levels according to the body hair density thereof and is displayed in a display mode indicating the level classified. It should be noted that the number of display modes is not limited to five and may be six or more. In FIG. 15, a higher density of dots (i.e., a darker color) indicates a greater body hair density. The display modes may be set so as to differ in density of dots (color intensity). Moreover, the display modes may be set so as to differ in color according to the hue value. When the display modes are set so as to differ in color according to the hue value, for example, a region having the highest body hair density may be shown in red and a region having the lowest body hair density may be shown in blue.

Then, as illustrated in FIG. 16, outputter 116 outputs display information 400 for displaying first heat map data 402 obtained from a first skin image and second heat map data 401 obtained from a second skin image in a state where first heat map data 402 and second heat map data 401 can be compared with each other.

### [Operation]

Next, an information processing method performed by information processing device 100 will be described.

FIG. 17 is a diagram illustrating an example of processing of capturing a skin image at a second timing (e.g., at the first time) and detecting a hair amount.

Information processing device 100 activates camera 107 and enters an image capturing preparation mode (S11).

Information processing device 100 determines whether the illuminance around camera 107 is appropriate for capturing an image of the face of a user, based on the result of detection performed by an image sensor of camera 107 (S12).

When information processing device 100 determines that the illuminance is appropriate (Yes in S12), the process proceeds to step S13, and when information processing device 100 determines that the illuminance is not appropriate (No in S12), the process returns to step S11. It should be noted that when the result of step S12 is No, information processing device 100 may present a message that prompts adjustment of the illuminance since the illuminance is not appropriate. The message may be displayed on display 106 or outputted as audio by a loudspeaker that is included in information processing device 100 and not shown in the Drawings. Moreover, when the result of step S12 is No, information processing device 100 may return to step S12.

Information processing device 100 determines whether the face size of the user detected by the image sensor of camera 107 is appropriate (S13).

When information processing device 100 determines that the face size is appropriate (Yes in S13), the process proceeds to step S14, and when information processing device 100 determines that the face size is not appropriate (No in S13), the process returns to step S11. It should be noted that when the result of step S13 is No, information processing device 100 may present a message that prompts adjustment of the distance between camera 107 of information processing device 100 and the face of the user since the face size is not appropriate. The message may be displayed on display 106 or outputted as audio by a loudspeaker that is included in information processing device 100 and not shown in the Drawings. Moreover, when the result of step S13 is No, information processing device 100 may return to step S13.

Information processing device 100 continuously captures multiple images (S14). Information processing device 100 continuously captures three images, for example. Thus, the three skin images in each of which the face of the user is captured are obtained.

Information processing device 100 performs count processing on a skin image having the highest resolution among the three skin images obtained in step S14 (S15). The details of the count processing will be described later with reference to FIG. 18.

Next, information processing device 100 determines whether to store the count result of the count processing (S16). Information processing device 100 may determine, for example, whether the count result falls within a predetermined range, and may determine to store the count result when the count result falls within the predetermined range and may determine not to store the count result when the count result falls outside the predetermined range.

When information processing device 100 determines to store the count result of the count processing (Yes in S16), the process proceeds to step S17, and when information processing device 100 determines not to store the count result (No in S16), the process returns to step S11.

Information processing device 100 stores the count result of the count processing in storage unit 117 (S17).

Information processing device 100 generates display information based on the count result (S18). The display information includes, for example, the count result. The display information may include the above-described heat map data.

Information processing device 100 outputs the display information (S19). Specifically, information processing device 100 displays the display information on display 106.

FIG. 18 is a flowchart illustrating the details of the count processing (S15). In step S15, steps S21 to S26 described below are performed.

Information processing device 100 sets, between the nose and the upper lip in the face of the user in skin image 200, target region 207 that is a target for body hair detection (S21).

Next, counter 112 of information processing device 100 binarizes skin image 200 for body hair detection (S22). Thus, binarized image 230 is obtained.

Next, information processing device 100 identifies, among the plurality of pixels constituting binarized image 230, pixels of the outline of a pixel group consisting of pixels each of which has a pixel value of 1, by searching for a pixel whose pixel value is 1. In other words, information processing device 100 identifies the outline of each body hair, based on binarized image 230 (S23).

Next, for each of a plurality of pixel groups each detected based on the outline thereof, information processing device 100 sets a rectangle that surrounds the outline of the pixel group (i.e., the outline of the body hair) and has the minimum area (S24).

Next, information processing device 100 identifies, as the length of the body hair, the long side of the rectangle set (S25).

Next, information processing device 100 counts, as the number of body hairs, the number of one or more rectangles each of which has the length of the body hair identified (i.e., the length of the long side of the rectangle) that falls within a predetermined range, among the plurality of rectangles (S26).

FIG. 19 is a diagram illustrating an example of processing of capturing a skin image at a first timing (e.g., at the second time or later) and detecting a hair amount.

Information processing device 100 activates camera 107 and enters an image capturing preparation mode (S21).

Information processing device 100 determines whether the illuminance around camera 107 is appropriate for capturing an image of the face of a user, based on the result of detection performed by an image sensor of camera 107 (S32).

When information processing device 100 determines that the illuminance is appropriate (Yes in S32), the process proceeds to step S33, and when information processing device 100 determines that the illuminance is not appropriate (No in S32), the process returns to step S31. It should be noted that when the result of step S32 is No, information processing device 100 may present a message that prompts adjustment of the illuminance since the illuminance is not appropriate. The message may be displayed on display 106 or outputted as audio by a loudspeaker that is included in information processing device 100 and not shown in the Drawings. Moreover, when the result of step S32 is No, information processing device 100 may return to step S32.

Information processing device 100 determines whether the face size of the user detected by the image sensor of camera 107 is appropriate (S33).

When information processing device 100 determines that the face size is appropriate (Yes in S33), the process proceeds to step S34, and when information processing device 100 determines that the face size is not appropriate (No in S33), the process returns to step S31. It should be noted that when the result of step S33 is No, information processing device 100 may present a message that prompts adjustment of the distance between camera 107 of information processing device 100 and the face of the user since the face size is not appropriate. The message may be displayed on display 106 or outputted as audio by the loudspeaker that is included in information processing device 100 and not shown in the Drawings. Moreover, when the result of step S33 is No, information processing device 100 may return to step S33.

Information processing device 100 continuously captures multiple images (S34). Information processing device 100 continuously captures three images, for example. Thus, the three skin images in each of which the face of the user is captured are obtained.

Information processing device 100 performs count processing on a skin image having the highest resolution among the three skin images obtained in step S34 (S35). The count processing is the same as that in FIG. 18.

Information processing device 100 compares (i) a first count result that is a result of the count processing performed on a first skin image captured at a first timing and (ii) a predicted hair amount value based on a second count result that is a result of the count processing performed on a second skin image captured at a second timing, and determines whether an absolute value of the difference between the first count result and the predicted hair amount value is less than or equal to a predetermined threshold value (S36).

When information processing device 100 determines that the absolute value is less than or equal to the predetermined threshold value (Yes in S36), the process proceeds to step S37, and when information processing device 100 determines that the absolute value is greater than the predetermined threshold value (No in S36), the process returns to step S31. It should be noted that, when the result of step S36 is No, information processing device 100 may present a message that prompts the user to recapture a skin image. The message may be displayed on display 106 or outputted as audio by the loudspeaker that is included in information processing device 100 and not shown in the Drawings.

Information processing device 100 calculates an evaluation value indicating a hair growth-inhibiting effect, based on the difference between the first count result and the predicted hair amount value (S37). It should be noted that the evaluation value may be calculated when the first count result and the second count result are compared in step S36.

Information processing device 100 stores the first count result of the count processing (S38).

Information processing device 100 generates display information based on the first count result and the second count result (S39). The display information includes, for example, the first count result and the second count result. Moreover, the display information may include the evaluation value calculated based on the difference between the first count result and the predicted hair amount value. As illustrated in FIG. 16, the display information may be display information 400 including: first heat map data 402 that is obtained from the first skin image; and second heat map data 401 that is obtained from the second skin image and disposed so that second heat map data 401 can be compared with first heat map data 402.

Information processing device 100 outputs the display information generated (S40). Specifically, information processing device 100 displays the display information on display 106.

### [Advantageous Effects, etc.]

Information processing device 100 according to the present embodiment is information processing device 100 that evaluates a hair growth-inhibiting effect of light-based hair removal device 10. Information processing device 100 includes obtainer 111, counter 112, and evaluator 113. Obtainer 111 obtains skin image 200 in which a target portion of a user (subject person) is captured. Counter 112 counts the number of body hairs from skin image 200. Evaluator 113 evaluates the hair growth-inhibiting effect, based on a result of comparison between (i) a first count result that is a result of counting performed on a first skin image captured at a first timing and (ii) a predicted hair amount value at the first timing based on a second count result that is a result of counting performed on a second skin image captured at a second timing that precedes the first timing by a first period.

According to this, since comparison is performed between (i) the first count result for the first skin image captured at the first timing and (ii) the predicted hair amount value predicted based on the hair growth-inhibiting effect due to a treatment performed at a timing corresponding to the second timing by light-based hair removal device 10, it is possible to determine whether a predicted hair growth-inhibiting effect has been realized. Thus, the hair growth-inhibiting effect of light-based hair removal device 10 can be appropriately evaluated.

Moreover, in information processing device 100 according to the present embodiment, the first timing is a timing after a second period elapses from when a treatment using light-based hair removal device 10 is performed. The second timing is a timing after the second period elapses from when an other treatment is performed subsequent to the treatment using light-based hair removal device 10.

Hence, since the first timing and the second timing can each be a timing after the second period elapses from when a corresponding treatment is performed, the hair growth-inhibiting effect can be evaluated in a way that reduces an error due to a body hair growth amount.

Moreover, in information processing device 100 according to the present embodiment, when the first count result is less than or equal to the predicted hair amount value, evaluator 113 calculates, based on a difference between the first count result and the predicted hair amount value, an evaluation value indicating the hair growth-inhibiting effect, and outputs the evaluation value calculated.

According to this, when the first count result is less than or equal to the predicted hair amount value, image capturing can be determined to be succeeded and the first count result obtained can be determined to be appropriate, and therefore an appropriate evaluation value can be calculated.

Moreover, in information processing device 100 according to the present embodiment, when the absolute value is greater than the predetermined threshold value, evaluator 113 outputs presentation information for prompting the user to capture a new skin image of the user (subject person).

According to this, when the absolute value is greater than the predetermined threshold value, image capturing can be determined to have failed, and therefore an appropriate first count result can be obtained by prompting image recapturing.

Moreover, in information processing device 100 according to the present embodiment, for each of a plurality of body hairs in skin image 200, counter 112 (i) extracts an outline of the body hair and (ii) sets a rectangle that surrounds the outline extracted and has a minimum area. Then, counter 112 counts, as the total number of body hairs, a total number of one or more rectangles each having at least one of a long side whose length falls within a predetermined range or a short side whose length falls within the predetermined range, among the rectangles set.

Hence, since a portion other than body hair, such as a skin wrinkle, a pore, or a mole, can be excluded from counting, the number of body hairs can be appropriately counted.

Moreover, in information processing device 100 according to the embodiment, counter 112 sets, between a nose and an upper lip of the user (subject person) in skin image 200, a target region that is a target for body hair detection, and sets the rectangles in the target region.

Hence, a facial hair can be appropriately counted as a body hair appearing on a human face.

Moreover, information processing device 100 according to the present embodiment further includes calculator 114, generator 115, and outputter 116. Calculator 114 divides, into a plurality of divided regions, a skin region that is a region of the target portion in skin image 200, and calculates a body hair density for each of the plurality of divided regions. Generator 115 generates heat map data 300 indicating the body hair densities in the skin region, based on a result of calculation performed by calculator 114. Outputter 116 outputs display information 400 for displaying first heat map data 402 obtained from the first skin image and second heat map data 401 obtained from the second skin image in a state where first heat map data 402 and second heat map data 401 can be compared with each other.

Hence, the distribution of the body hair densities can be visualized.

### [Variations]

(1) Although information processing device 100 includes camera 107 in the above-described embodiment, the present disclosure is not limited to this example. Information processing device 100 does not necessarily include camera 107 as long as information processing device 100 can obtain (receive) a skin image from another information processing device via communication IF 104. For example, information processing device 100 may obtain a skin image from a digital camera or from an information terminal having a camera function, such as a smartphone or a tablet device, or may obtain a skin image from another information processing device in which the skin image is stored. In such a case, obtainer 111 may be realized by communication IF 104.
(2) Although a facial hair has been exemplified as a body hair in a target portion in the above-described embodiment, the present disclosure is not limited to this example and a body hair in a target portion may be a hair in another portion. For example, a body hair in a target portion may be a body hair growing on any of an underarm, a chest, a hand, a bikini area, or a leg.
(3) Although evaluator 113 outputs presentation information by causing outputter 116 to display the presentation information in the above-described embodiment, the present disclosure is not limited to this example and evaluator 113 may output presentation information by transmitting the presentation information to a mobile terminal of a user. The mobile terminal may display the presentation information upon receipt of the presentation information. Thus, the presentation information displayed on the mobile terminal can be visually recognized by the user, and the user can be prompted to capture a skin image of the user, using information processing device 100.

### [Others]

It should be noted that in the above-described embodiment, each constituent element may be configured in the form of an exclusive hardware product, or may be realized by executing a software program suitable for the constituent element. Each constituent element may be realized by a program executing unit, such as a CPU or a processor, loading and executing a software program recorded on a recording medium such as a hard disk or a semiconductor memory. Here, software that realizes an information processing device according to the above-described embodiment or the like is a program for causing a computer to execute each step included in the flowcharts illustrated in the Drawings.

It should be noted that the following cases are included in the present disclosure.
(1) Each device described above is, specifically, a computer system including a microprocessor, a ROM, a RAM, a hard disk unit, a display unit, a keyboard, a mouse, etc. A computer program is stored in the RAM or the hard disk unit. Each device achieves its function as a result of the microprocessor operating according to the computer program. Here, the computer program includes a combination of a plurality of instruction codes indicating commands to a computer for achieving a given function.
(2) Some or all of the constituent elements of each device described above may be implemented as a single system large scale integration (LSI). The system LSI is a super-multifunction LSI manufactured by integrating a plurality of components on a single chip, and specifically is a computer system including a microprocessor, a ROM, a RAM, etc. A computer program is stored in the RAM. The system LSI achieves its function as a result of the microprocessor operating according to the computer program.
(3) Some or all of the constituent elements of each device described above may be implemented as an IC card or a single module, each detachable from and attachable to the device. The IC card or the module is a computer system including a microprocessor, a ROM, a RAM, etc. The IC card or the module may include the super-multifunction LSI described above. The IC card or the module achieves its function as a result of the microprocessor operating according to a computer program. The IC card or the module may be tamperproof.
(4) The present disclosure may be implemented as the above-described method. Moreover, the present disclosure may also be implemented as a computer program realizing the method using a computer, or as a digital signal including the computer program.

Moreover, the present disclosure may also be implemented as a computer-readable recording medium having recorded thereon the computer program or the digital signal, such as a flexible disk, a hard disk, a CD-ROM, an MO, a DVD, a DVD-ROM, a DVD-RAM, a Blu-ray (registered trademark) Disc (BD), or a semiconductor memory. Furthermore, the present disclosure may also be implemented as the digital signal recorded on the above mentioned recording medium.

Furthermore, the present disclosure may transmit the computer program or the digital signal via a telecommunication line, a wireless or wired communication line, a network represented by the Internet, a data broadcast, or the like.

Furthermore, the present disclosure may be a computer system including a microprocessor and a memory, the memory may store the computer program, and the microprocessor may operate according to the computer program.

Furthermore, the present disclosure may be implemented by another independent computer system by transferring the program or the digital signal recorded on the recording medium or transferring the program or the digital signal via the network or the like.

(5) The above-described embodiment and the above-described variations may be implemented in any combination.

### [Industrial Applicability]

The present disclosure is useful as an information processing device or the like that can appropriately evaluate a hair growth-inhibiting effect of a light-based hair removal device.

### [Reference Signs List]

- 10: light-based hair removal device
- 100: information processing device
- 101: processor
- 102: main memory
- 103: storage
- 104: communication IF
- 105: input device
- 106: display
- 107: camera
- 111: obtainer
- 112: counter
- 113: evaluator
- 114: calculator
- 115: generator
- 116: outputter
- 117: storage unit
- 200: skin image
- 201 to 206: feature point
- 207: target region
- 208, 231: region
- 210, 221: block
- 220: processing-target pixel
- 230: binarized image
- 240: circle
- 241: pixel group
- 242, 243: rectangle
- 300: heat map data
- 400: display information
- 401: second heat map data
- 402: first heat map data
- A1: region
- L1 to L3: straight line
- L4: polygonal line
- Th, Th1, Th2: threshold value
- c: constant

## Claims

1. An information processing device that evaluates a hair growth-inhibiting effect of a light-based hair removal device, the information processing device comprising:
an obtainer that obtains a skin image in which a target portion of a subject person is captured;
a counter that counts a total number of body hairs from the skin image; and
an evaluator that evaluates the hair growth-inhibiting effect, based on a result of comparison between (i) a first count result that is a result of counting performed by the counter on a first skin image captured at a first timing and (ii) a predicted hair amount value at the first timing based on a second count result that is a result of counting performed by the counter on a second skin image captured at a second timing that precedes the first timing by a first period, the first skin image and the second skin image each being the skin image.

2. The information processing device according to claim 1, wherein
the first timing is a timing after a second period elapses from when a treatment using the light-based hair removal device is performed, and
the second timing is a timing after the second period elapses from when an other treatment is performed subsequent to the treatment using the light-based hair removal device.

3. The information processing device according to claim 1 or claim 2, wherein
when an absolute value of a value obtained by subtracting the first count result from the predicted hair amount value is less than or equal to a predetermined threshold value, the evaluator calculates, based on a difference between the first count result and the predicted hair amount value, an evaluation value indicating the hair growth-inhibiting effect, and outputs the evaluation value calculated.

4. The information processing device according to claim 3, wherein
when the absolute value is greater than the predetermined threshold value, the evaluator outputs presentation information for prompting the subject person to capture a new skin image of the subject person.

5. The information processing device according to any one of claim 1 to claim 3, wherein
the counter:
for each of a plurality of body hairs in the skin image, (i) extracts an outline of the body hair and (ii) sets a rectangle that surrounds the outline extracted and has a minimum area; and
counts, as the total number of body hairs, a total number of one or more rectangles each having at least one of a long side whose length falls within a predetermined range or a short side whose length falls within the predetermined range, among the rectangles set.

6. The information processing device according to claim 5, wherein
the counter sets, between a nose and an upper lip of the subject person in the skin image, a target region that is a target for body hair detection, and sets the rectangles in the target region.

7. The information processing device according to any one of claim 1 to claim 3, further comprising:
a calculator that divides, into a plurality of divided regions, a skin region that is a region of the target portion in the skin image, and calculates a body hair density for each of the plurality of divided regions;
a generator that generates heat map data indicating the body hair densities in the skin region, based on a result of calculation performed by the calculator; and
an outputter that outputs display information for displaying first heat map data obtained from the first skin image and second heat map data obtained from the second skin image in a state where the first heat map data and the second heat map data can be compared with each other, the first heat map data and the second heat map data each being the heat map data.

8. An information processing method that is performed by an information processing device that includes one or more processors and evaluates a hair growth-inhibiting effect of a light-based hair removal device, the information processing method comprising the following performed by the one or more processors:
obtaining a skin image in which a target portion of a subject person is captured;
counting a total number of body hairs from the skin image; and
evaluating the hair growth-inhibiting effect, based on a result of comparison between (i) a first count result that is a result of the counting performed on a first skin image captured at a first timing and (ii) a predicted hair amount value at the first timing based on a second count result that is a result of the counting performed on a second skin image captured at a second timing that precedes the first timing by a first period, the first skin image and the second skin image each being the skin image.

9. A program for causing a computer to execute the information processing method according to claim 8.
